# EUROPEAN PATENT SPECIFICATION

(11) **EP 1 810 998 B1**
(45) Date of publication and mention of the grant of the patent: **12.03.2014**
(21) Application number: 07000988.1
(22) Date of filing: 18.01.2007
(51) Int. Cl.: C09B 23/08, G01N 33/58

(54) **Fluorescent cyanine dye**
Fluoreszierender Cyaninfarbstoff
Colorant Cyanine Fluorescent

(30) Priority: 19.01.2006 IT SV20060002
(43) Date of publication of application: 25.07.2007
(73) Proprietor: Ferrania Technologies S.p.A., 17014 Cairo Montenotte, SV (IT)
(72) Inventor: Puppo, Paola, 17014 Cairo Montenotte (SV) (IT); Cogliolo, Isabella, 17014 Cairo Montenotte (SV) (IT)
(74) Representative: Allaix, Roberto

(56) References cited:
- EP-A- 0 350 026
- EP-A- 1 065 250
- EP-A- 1 152 008
- WO-A-96/10213
- WO-A-97/13810
- WO-A1-00/16810
- US-A- 5 679 516
- US-A- 5 958 667
- US-A- 5 968 479
- US-A- 6 159 657
- US-A1- 2006 269 926
- US-B1- 6 593 148
- DATABASE WPI Week 200634 Thomson Scientific, London, GB; AN 2006-318546 XP002504203 -& CN 1 702 118 A (UNIV DALIAN TECHNOLOGY) 30 November 2005 (2005-11-30)

## Description

This invention relates to the field of labeling chemistry including labeling reagents, processes for target labeling, labeled targets, processes for preparing labeling reagents, and the like. This invention also relates to the use of such compositions and processes in other processes for nucleic acid and enzymatic activity determinations and analyses.

### Background of the art

Fluorescent dyes are widely used for biological, biochemical or chemical applications in which a highly sensitive detection reagent is desirable. Molecules labeled with fluorescent dye reagents enable the researcher to determine the presence, quantity or location of such molecules by monitoring their fluorescence. The quenching and energy transfer properties of fluorescent dyes also render the dyes useful for a variety of methods permitting investigators to monitor the in vivo and in vitro interactions between labeled molecules (e.g., protein:protein, protein:nucleic acid, nucleic acid:nucleic acid, and protein: or nucleic acid: interactions with drugs, drug candidates or other chemical entities). Fluorescent dyes are also useful for non-biological applications, such as photographic media.

In comparison to radiolabels frequently used for similar purposes, fluorescent dyes have the advantages of being safe to handle and dispose of and relatively stable. In addition, fluorescent dyes are detectable in real time, and the possibility of exploiting dyes with different fluorescence excitation and emission spectra permits differential detection of two, three or more different labeled species in a given mixture.

There is a wide variety of fluorescent dyes known and used in various fields. One class, the cyanine dyes, is very frequently used in biological and biochemical applications. Cyanine dyes are generally characterized by the presence of a pair of nitrogen-containing heterocycles ("terminal heterocycles") connected by a polymethine bridge over which bond resonance occurs. Most cyanine dyes exhibit high visible absorbance and reasonable resistance to photodegradation.

The arylsulfonate cyanine dyes developed by Waggoner et al. (U.S. Pat. Nos. 5,268,486; 5,486,616; 5,569,587 5,569,766; 5,627,027 and 6,225,050) provide higher levels of solubility in aqueous media than similar hydrophobic materials. These dyes, with their sulfonate groups are also less prone to hydrophobic interactions such as stacking interactions when covalently bound to macromolecules. The latter effect leads to higher quantum yields in aqueous media and translates to a brighter labeling reagent, an important criteria for sensitive detection methods that use fluorescence. These arylsulfonate cyanine dyes also have functional groups appended to them for covalently coupling to macromolecules. Their absorption wavelength can be tailored to match existing instrumentation, which in turn increases their potential to be used as fluorophores for multiparameter analysis in cytometry and diagnostics.

U.S. Pat. No. 5,268,486 and US 5,486,616 disclose water soluble cyanine dyes comprising a sulfonic acid or a sulfonate moyety and which contain reactive moieties including isothiocyanate, isocyanate, monochlorotriazine, dichlorotriazine, mono- or di-halogen substituted pyridine, mono- or di-halogen substituted diazine, maleimide, aziridine, sulfonyl halide, acid halide, hydroxysuccinimide ester, hydroxysulfosuccinimide ester, imido ester, hydrazine, azidonitrophenyl, azide, 3-(2-pyridyl dithio)-proprionamide, glyoxal and aldehyde. These dyes are chosen to label target molecules containing amino-, hydroxy- and sulfhydryl groups.

US 5,569,587 discloses a method for labeling a component of an aqueous liquid, such as a protein, by reacting a luminescent cyanine dye having a reactive substituent (e.g., active ester, isothiocyanate, iodoacetyl and the like) and at least one sulfonate group or sulfonic acid group attached to the aromatic nucleus.

US 5,569,766 discloses a cyanine type luminescent dye comprising a five or six membered heterocycle ring comprising nitrogen and sulfur and a sulfonic acid or sulfonate moiety attached to the aromatic ring.

U.S. Pat. No. 5,627,027 and US 6,225,050 describe a method for labeling proteins, cells, nucleic acid and DNA with a cyanine dye, as well as the reaction therebetween. The cyanine dye specified is limited in the position and identity of a reactive group within the dye. The reactive group is limited to isothiocyanate, isocyanate, monochlorotriazine, dichlorotriazine, mono- or di-halogen substituted pyridine, mono- or di-halogen substituted diazine, aziridine, sulfonyl halide, acid halide, hydroxy succinimide ester, hydroxy sulfosuccinimide ester, imido ester, glyoxal and aldehyde. The method still further requires forming a covalent bond between the reactive group on the dye, and an amine or hydroxyl group on the material being labeled utilizing an electrophilic mechanism for coupling the dye and material being labeled.

WO 03/074091 discloses a near infrared fluorescent contrast agent. In the specification there is no mention to sulfonamide substitution/s to the aromatic ring.

EP 1,221,465 describes a symmetric cyanine dye having formula: 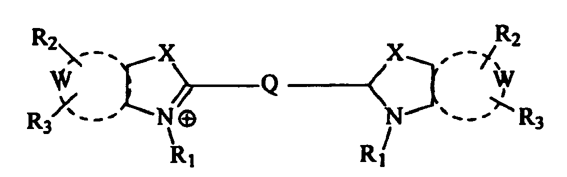 where R₂ and R₃ are independently selected from the group consisting of hydrogen atom, a sulphonic moiety and a sulphonate moiety. In the specification there is no mention to sulfonamide substitution/s to the aromatic ring.

EP 1,065,250 discloses a fluorescent compound wherein attached to the aromatic ring there is a reactive substituted alkyl derivative having formula -SO₂NH(CH₂)ₘW(CH₂)ₙZ, where W is absent or is a group selected from -SO₂NH-, -O-, -COO- or -CONH-; n=0-12 and m=0-12 with the proviso that the sum of m and n is equal to or lower than 12 and at least one of m and n is different from zero and Z is a N, O or S nucleophile functionality or is a functionality capable of reacting with N, O, S nucleophiles. The -SO₂NH(CH₂)ₘW(CH₂)ₙZ group is a reactive spacer link "chosen because it does not interfere with the fluorescent behaviour of the dye and also because its precursor, the sulfonate group is commonly found in a very large number of arylamines which are needed for the synthesis of the cyanine dyes" (lines 57-58, page 2).

EP1,090,961 discloses an unsymmetrical indolenin pentamethine cyanine dye having a sulfonamido group mentioned among the possible substituents of the aromatic ring. The application for the claimed class of dye is optical recording media and the advantages of the claimed structure consist in excellent recording features when used in high-speed-recordable-type optical recording media. These features are due to the fact that all the claimed dyes have a decomposition point very close or undistinguishable respect to the melting point. No reference to photophysical properties are reported and moreover the disclosed dyes do not have a reactive group, as defined in the following table 1, for the conjugation with the biological substrate.

EP 1 152 008 provides a fluorescent nucleotide represented by the formula: A-B-C, wherein A represents a residue of natural or synthetic nucleotide, oligonucleotide, polynucleotide or derivative thereof and binds B at a base moiety in said residue; B represents a divalent linking group or a single bond; and C represents a monovalent group derived from a fluorescent dye having 0 or 1 sulfonic acid group or phosphoric acid group in a molecule. Fluorescent nucleotides are useful for labeling nucleic acids.

US 2006/0269926 relates to the filed of labeling compositions, reagents and processes that are useful in applications related to detection, quantification and localization of target molecules of interest that include nucleic acids and protein.

WO 00/16810 discloses a near infrared fluorescent contrast agent comprising a compound having three or more sulfonic acid groups in a molecule and a method of fluorescence imaging comprising introducing said agent into a living body, exposing the body to an excitation light and detecting near infrared fluorescence from the contrast agent.

CN1702118 relates to a near-infrared methenyl fluorescence dye, which is made by displacement at midposition of methine colors by nucleophilic reagent containing nitrogen and sulfur and can be used as high fidelity fluorescence labeling probe of biomolecules such as protein, sugar and DNA.

Cyanine and related polymethine dyes having light absorbing properties, have been employed for many years in photographic films as sensitizer dyes. Photographic sensitizer dyes extend the spectral sensitivity of silver halide grains. Sensitizer dyes require light absorbing properties, but they do not necessarily require luminescence (fluorescence or phosphorescence) properties.

In more recent years, there has been an upsurge of new use of fluorescent polymethine dyes as fluorescent markers in innovative technological areas, such as laser and electro-optic applications, optical recording media, medical, biological and diagnostic. Fluorescent polymethine dyes have many advantages over traditional fluorescent labels, such as fluorescein or rhodamines, as their absorption wavelength can be easily changed according to the change of the number of methine groups and/or substituent/s and they can be easily efficiently excited by means of small, inexpensive solid state device such as laser diodes or light emitting diodes, with extinction coefficient often several times higher than fluorescein and rhodamines.

To be useful as a fluorescent markers, the polymethine cyanine dyes must have a suitable functional group to make the covalent bond with substrate possible. For example if the fluorescent dye has to be conjugated with a protein, the functional group present in the dye has to be covalently reactive with amine, hydroxyl and/or sulfhydril groups.

In dyes with carboxyl groups, the latter can be converted into their N-succinimide-esters, which then react directly with amino groups on the biomolecule or via a so-called amino linker with hydroxyl groups. In dyes with alkyl hydroxy groups, the latter can be converted into phosphoramidites, which react directly with the free 5'-hydroxy group of nucleotides to form a phosphite bond, which subsequently oxydises to form a stable phosphate bond (US 6,734,310). Some examples are reported in Table 1 (EP1, 086, 179):

**TABLE 1**

| *Reactive groups* *(Fluorescent dye)* | *Functional Groups* *(Substrate)* |
|---|---|
| Succinimidyl | Primary amino, secondary amino |
| Anhydrides, acid halides | Primary amino, secondary amino, hydroxyl |
| Isothiocyanate | Amino groups |
| Vinylsulphone | Amino groups |
| Dichlorotriazines | Amino groups |
| Haloacetamides, maleimides | Thiols, imidazoles, hydroxyl, amines |
| Carboxyl | Amino, hydroxyl, thiols |
| Phosphoramidites | Hydroxyl groups |

One important characteristic of these dyes is photostability. Under irradiation the fluorophore may decrease the intensity of fluorescence emission. This phenomenon, known as photobleaching of the fluorophore, is detrimental and makes the fluorophore not useful for the application where the output is the fluorescence intensity of the molecule.

For this reason there is a continuous search of new structures able to provide good photostability.

### SUMMARY OF THE INVENTION

The dyes of the present invention are a novel class of fluorescent compounds with formula I: 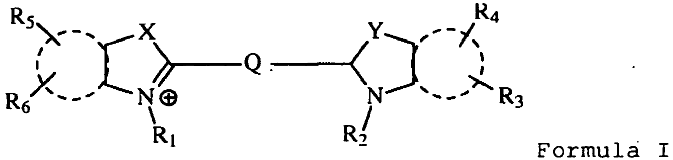 wherein:
X and Y are both -C(CH₃)₂;
the dotted curves independently represent the carbon atoms required to form a benzo-condensed or a naphtho-condensed ring;
R₁ and R₂ each independently represents sulfoalkyl, carboxyalkyl or sulfatoalkyl groups, wherein the alkyl chain of said R1 and R2 groups comprises from 1 to 5 carbon atoms;
R₃, R₄, R₅ and R₆ each independently is hydrogen atom, halogen atom, alkyl group, alkoxy group, amino group, and sulfo group;
wherein
at least one of R₃, R₄, R₅ and R₅ is a -SO₂NR₇R₈ group wherein R₇ and R₈ each independently represents hydrogen, unsubstituted alkyl, aryl, or -COCH₃, with the proviso that when R₇ is -COCH₃, R₈ is hydrogen;
Q is a polymethine chain having from 3 to 7 carbon atoms selected from the group consisting of: 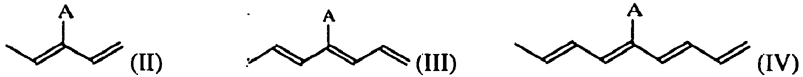 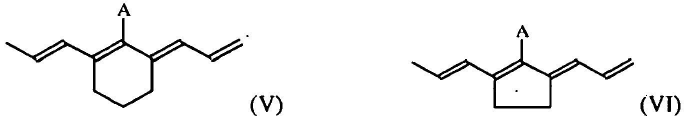 wherein A represents hydrogen, halogen, alkyl, aryl, cyano, -N(R₉R₁₀) where R₉ and R₁₀ each independently represents alkyl, aryl or together represent the non-metal atoms necessary to form a 5- or 6-membered heterocyclic ring, or -Z-R₁₁ where Z is O or S, and R₁₁ represents alkyl or aryl;
at least one of R₁, R₂, A comprises a substituent selected from the group consisting of succinimidyl, anhydrides, acid halides, isothiocyanate, vinylsulphone, dichlorotriazines, haloacetamides, maleimides, carboxyl, phosphoramidites.

The above reported structure shows an improved photostability when compared to commercial reference molecules.

### DETAILED DESCRIPTION OF THE INVENTION

In the above mentioned formula, X and Y are both represented by the -C(CH₃)₂ group.

In the above mentioned formula, R₁ and R₂ groups are represented by an alkyl chain comprising from 1 to 5 carbon atoms.

In the above mentioned formula, R₇ and R₈ are preferably both hydrogen.

In the above mentioned formula, the 5- or 6-membered heterocyclic ring is preferably selected from the group consisting of piperidine, piperazine, piperimidine, piperidazine, morpholine, pyrroiidine, imidazole and triazole.

### Method of Preparation

The dyes of this invention are prepared as follows:
The synthesis of the below reported dyes are well known and described in the literature (Hamer, Cyanine Dyes and Related Compounds, Interscience 1964; Mujumdar et al., Cytometry 10:11-19, 1989; Mujumdar et al., Bioconjugate chemistry, Vol. 4, number 2, US patent nr. 4,062,682).

### Synthesis of Intermediate I (2,3,3-trimethyl-5-sulphonamido-indolenine)

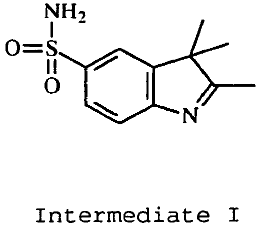

2,3,3-trimethyl-5-sulphonamide-indolenine is prepared as described in US patent No. 4,062,682, column 11, lines 30-40, with a yield of 92%.

### Synthesis of intermediate II (1-δ-sulphoethyl,-2,3,3-trimethyl-5-sulphonamido indoleninium inner salt)

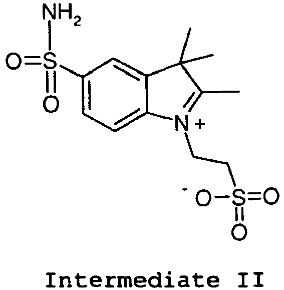

150 g of 2-bromoethanesulfonic acid sodium salt, Fluka, 1,5 1 of ethanol are mixed together, the mixture is then cooled to 5°C and 29,2 g of hydrochloric acid gas are bubbled through the mixture. After that, the mixture is brought back to RT and left under stirring for 4 hours. After filtration of sodium chloride, the mixture is concentrated to dryness under vacuum.

1,6 g of 2-bromoethanesulfonic acid, obtained with the method reported above, 1,3 g of intermediate I, 0,7 g of triethylamine and 5 ml of butirronitrile are mixed together and heated to reflux for 20 hours. The mixture is then cooled to room temperature and 15 ml of acetone are added. The solid product is vacuum filtered and washed with methanol and acetone. The reaction yield is 50%.

### Synthesis of intermediate III (1-ε-carboxyethyl,-2,3,3-trimethyl-5-sulphonamido indoleninium bromide salt)

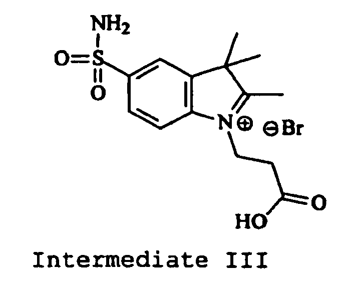

21,85 g (0,092 moles) of intermediate I , 15,3 g (0,100 moles) of 3-bromopropionic acid, Aldrich, are mixed in 36,1 g of CH₃CN and heated to reflux for 17 hours. The reaction mixture is then cooled to RT and vacuum filtered. The solid product is washed on the filter with CH₃CN and then dried. The reaction yield is 70%.

### Synthesis of Intermediate IV(1-δ-sulfobutyl-2,3,3-trimethylindoleninium inner salt)

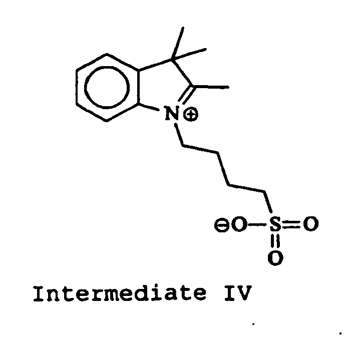

This synthesis was carried out according to the method reported in "Cyanine Dye Labeling Reagents Containing Isothiocyanate Groups", R. B. Mujumdar et al., Cytometry 10:11-19 (1989), with yield 80%.

### Synthesis of Intermediate V

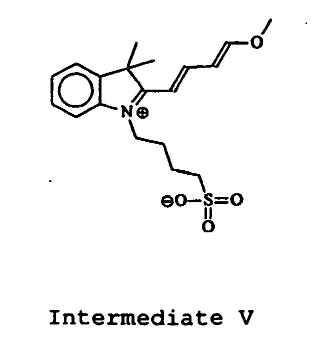

This synthesis was carried out according to the method reported in "Cyanine Dye Labeling Reagents Containing Isothiocyanate Groups", R. B. Mujumdar et al., Cytometry 10:11-19 (1989), with yield 90%.

### Synthesis of Dye A, invention

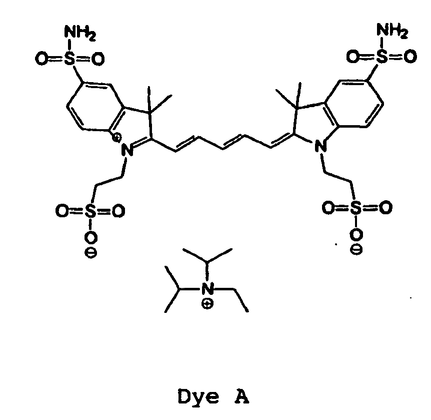

1,88 g of intermediate II (0,0054 moles), 0,78 g of malonaldheyde dianil hydrochloride (0,0030 moles), prepared from malonaldehyde bis(dimethylacetal) and aniline according to method described in US2,549,097, 12 ml of EtOH, 0,84 g of N-ethyl diisopropylamine (0,0065 moles) and 1,94 g of acetic anhydride are mixed together and heated to reflux for 30 minutes. The reaction mixture is then cooled to RT and vacuum filtered. The solid product is washed on the filter with EtOH and then dried. The reaction yield is 85%.

### Synthesis of Dye B, invention

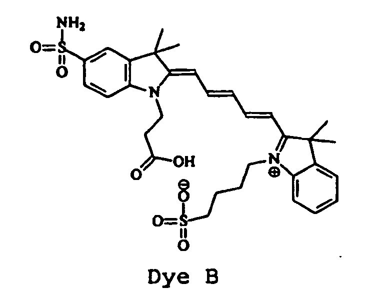

2,0 g of intermediate III (0,0051 moles) and 1,86 g of intermediate V (0,0051 moles), 50 ml of EtOH and 1,00 g of CH₃COONa are mixed together and heated to reflux for 30 minutes. The reaction mixture is then cooled to RT and vacuum filtered. The solid product is washed on the filter with EtOH and AcOEt and then dried. The reaction yield is 88%.

2,98 g of crude dye are dissolved in 48,6 g of deionized water at a temperature of 60°C; under stirring 1,1 g of a water solution of HCl, 3,6% w/w are added. The mixture is then cooled to RT and vacuum filtered. The product is washed with deionized water and acetone. The yield is 56%. The product has a purity >98%, tested by HPLC both @280 and @650 nm.

### Synthesis of Dye C, comparison

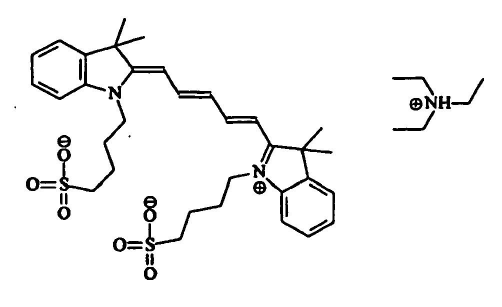

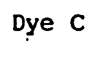

2,0 g of intermediate IV (0,0068 moles) and 0,96g of malonaldehyde dianil hydrochloride (0,0037 moles), prepared from malonaldehyde bis(dimethylacetal) and aniline according to method described in US2,549,097, 2,42 g of acetic anhydride, 0,82 g of triethylamine (0,0080 moles) and 15 ml of EtOH are mixed together and heated to reflux for 30 minutes. The reaction mixture is then cooled to RT and the dye is precipitated by adding ethyl acetate (about 50 g). The product is then vacuum filtered, washed with ethyl acetate and dried. The reaction yield was 81%.

The dye was further purified by a cristallization step in ethanol.

### Cy5^{TM}, Amersham Biosciences, reference

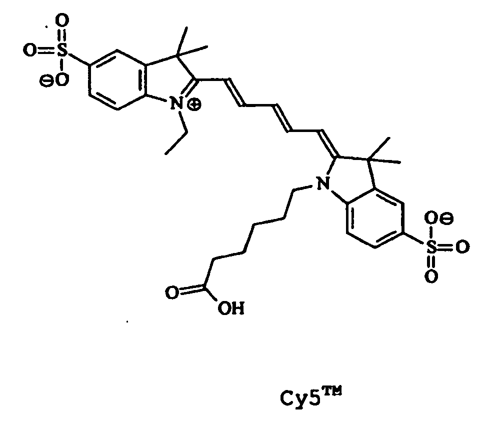

### Synthesis of Intermediate VI (2,3,3-trimethyl-sulfoindolenine, sodium salt)

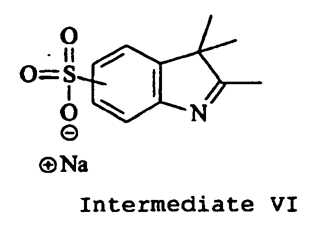

50,0 g (0,314 moles) of 2,3,3-trimethylindolenine (Aldrich) are added dropwise to 200 ml of fumed sulfuric acid (20% free SO₃), while the reaction mixture is cooled in an ice-water bath. At the end of the addition the system is kept at room temperature for one hour. The reaction mixture is then poured into 1 Kg of ice and neutralized with about 350 ml of an aqueous solution of sodium hydroxide, 30% w/w. The precipitated salt is filtered away and the mother liquor, containing the product, is vacuum evaporated to dryness. The so obtained crude product is dissolved in methanol, the residual salts are filtered away and the solution is again vacuum evaporated to dryness. The product is dispersed with about 50-100 ml of acetonitrile, is filtered under vacuum and washed on filter with acetonitrile. The solid obtained is dried in a vacuum oven at 40°C. The final yield is 78%.

### Synthesis of intermediate VII (1-δ-sulfobutyl-2,3,3-sulfotrimethyl indoleninium, inner salt)

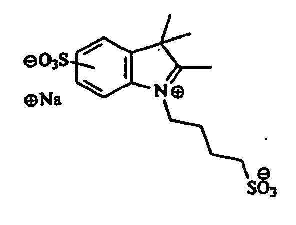

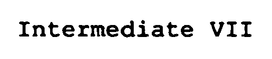

30 g (0,115 moles) of intermediate VI, 18,8 g (0,138 moles) of butansultone (Aldrich) and 10 ml of butyronitrile are heated to reflux and kept at reflux for 24 hours. The reaction mixture is then cooled to room temperature and 150 ml of acetone are added. The system is left under stirring at room temperature for about 8-10 hours. The reaction mixture is then filtered under vacuum and the solid is redispersed in acetone and then it is again filtered under vacuum and washed with acetone. The solid is dried in a vacuum oven vacuum at 40°C. The final yield is 95%.

### Synthesis of intermediate VIII (1-ε-carboxypentyl-2,3,3-trimethyl-5-sulphonamido indoleninium, bromide salt)

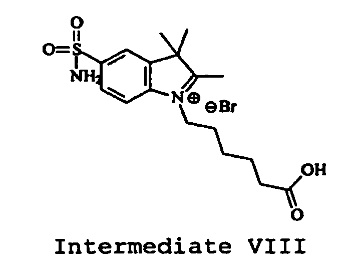

20,0 g (0,084 moles) of intermediate I, 16,4 g (0,084 moles) of 6-bromohexanoic acid (Aldrich) are mixed in 10 ml of butyronitrile and heated to reflux for 17 hours. The reaction mixture is then cooled to room temperature, 30 ml of acetone are added. After having left the reaction mixture under stirring for about 2 hours, it is filtered under vacuum and the solid product is washed on the filter several times with acetone.The so obtained crude product is then dispersed in about 200 ml of acetonitrile and heated to reflux for one hour. The reaction mixture is cooled to room temperature and the solid is filtered under vacuum and washed with acetonitrile and acetone. The solid product is then dried in a vacuum oven at 40°C. The final yield is 70%.

### Synthesis of intermediate IX

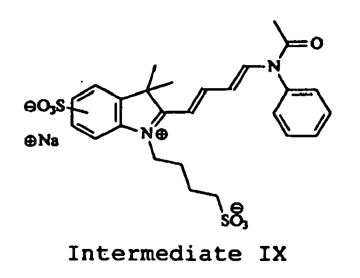

10,0 g (0,0252 moles) of intermediate VII, 7,2 (0,0277 moles) of malonaldehyde dianil hydrochloride, prepared from malonaldehyde bis(dimethylacetal) and aniline according to method described in US2,549,097, 40 ml di acetic acid and 40 ml of acetic anhydride are kept at reflux for 30 minutes. The reaction mixture is then cooled and poured in 1 l of ethyl acetate and kept under stirring at room temperature for one hour. The solid product is filtered under vacuum and washed with ethyl acetate and dried in a vacuum oven at 35°C. The yield is 70%.

### Synthesis of dye D, invention

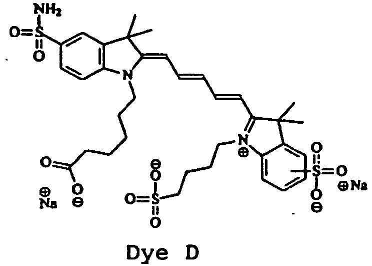

6,0 g of intermediate IX (0,0105 moles) and 5,5 g of intermediate VIII (0,0126 moles), 60 ml of ethanol and 2,1 g of CH₃COONa are mixed together and heated to reflux for 30 minutes. The reaction mixture is then cooled to room temperature and vacuum filtered. The solid product is washed on the filter with EtOH and then dried in a vacuum oven at 40°C. The yield is 70%.

The so obtained crude dye is purified by HPLC (reverse phase, eluent: water-acetonitrile, buffer 0.1M triethylammonium bicarbonate). The purified dye has an area % >98%, by HPLC.

### Synthesis of Intermediate X

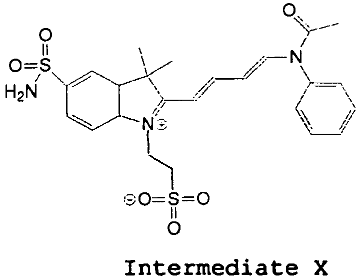

10,0 g (0,0289 moles) of Intermediate II, 8,2 g (0,0317 moles) of malonaldehyde dianil hydrochloride, prepared from malonaldehyde bis(dimethylacetal) and aniline according to method described in US2,549,097, and 40 ml of acetic acid are mixed together and heated to reflux for 3 hours. The reaction mixture is then cooled down to room temperature and poured in about 500 ml of acetone. The solid is vacuum filtered and washed on filter with acetone. The solid is dried in a vacuum oven at 35°C. The yield is 70%.

### Synthesis of dye E, invention

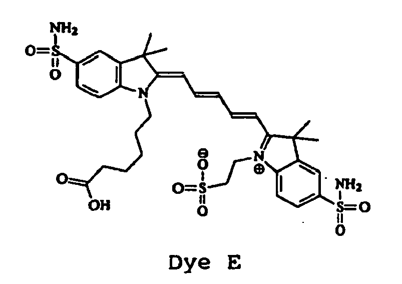

5,0 g of intermediate X (0,0105 moles) and 5,5 g of intermediate VIII (0,0126 moles), 50 ml of ethanol and 2,1 g of CH₃COONa are mixed together and heated to reflux for 1 hour. The reaction mixture is then cooled to room temperature and vacuum filtered. The solid product is washed on the filter with EtOH and then dried in a vacuum oven at 40°C. The yield is 70%.

0,70 g of crude dye are dissolved in 20 ml of deionized water at a temperature of 60°C; under stirring about 3 ml of a solution of HCl, 3,6% w/w are added. The mixture is then cooled to room temperature and vacuum filtered. The product is washed with deionized water and acetone. The yield is 50%.

The so obtained dye is further purified by HPLC (reverse phase, eluent: water-acetonitrile, buffer 0.1M triethylammonium bicarbonate). The purified dye has an area % >98%, by HPLC.

The dyes of the invention are conjugated with ligands which comprise the first element of one or more specific binding pairs, with the second element of the pairs being related to the analyte(s) of interest.

Most commonly, the second element of the specific binding pair and the analyte to which it is attached are present in an aqueous environment. In this case, if the dye itself is water soluble it is most common for the dye to include a linking group for covalently bonding to the first element of the specific binding pair. Usually, the dye includes a single binding group. These groups may be attached to the heterocyclic group through one of its R groups, or its nitrogen.

In another embodiment, the fluorescent dye may be attached to or encapsulated within a particle. In this case, the particle may have water-solubilizing groups attached to it and may provide the necessary groups for binding to the first elements of the specific binding pairs. In this case, the dye plus particle is considered to be the dye-ligand conjugate.

The dyes of this invention and their dye-ligand conjugates can be employed as elements of signal producing systems. These systems include a sample zone in which a sample suspected of including one or more analytes is located. The dye-ligand conjugates are contacted with the sample in the zone to form one or more specific binding pairs between analytes and dye-ligand conjugates.

A source of electromagnetic radiation, typically a laser irradiates the sample and emission from the dye-ligand conjugate is detected. The art teaches a great number of specific binding pairs based on assay techniques employing prior fluorescent dyes. These assay techniques may be employed with the novel fluorescent dyes provided by the present invention.

The dyes of the invention can be conjugated to members of a specific binding pair by techniques that are known in the art. The members of the specific binding pair are referred to as ligand and receptor (antiligand). These will usually be members of an immunological pair such as antigen-antibody, although other specific binding pairs such as biotin-avidin, hormone-hormone receptors, nucleic acid duplexes, IgG-protein A, DNA-DNA, DNA-RNA, and the like, that are not typically classed as immunological pairs are included in the definition of specific binding pair. Such conjugation can be the result of direct bond formation between the dye and the binding pair member. On the other hand, a linking group can be introduced into the dye or the binding pair member for attachment to the other component. A functionality for attachment such as carboxylic acid, hydroxyl, thio, amino, aldehydic, amido, activated ethylenes such as maleimide, sulfonic acids, and the like can be introduced into the dye or the binding pair member if such functionality is not originally present in the dye or the binding pair member. Methods of conjugation involving binding pair members are described in e.g., U.S. Pat. No. 3,817,837.

The compounds of the invention have properties that are very desirable for their use in assays. The compounds have high extinction coefficients, high quantum efficiencies, chemical stability, and satisfactory Stokes shifts. In the assay a fluorescent compound is employed to generate a signal in relation to the presence or amount of the material of interest in the sample and an energy source for excitation of the fluorescent compound is also employed. The improvement in such assay comprises employing a dye of the invention, particularly a water soluble dye, as the fluorescent compound and a helium/neon laser as the energy source. The dyes can be rendered water soluble by incorporating a group or functionality imparting water solubility into the dye. Alternatively, the dye can be rendered water compatible by employing surfactants, such as, for example, Triton X-100 or sodium dodecyl sulfate, cyclodextrin or the like, in the assay medium. In another alternative, latex particles or particles such as liposomes, cells and the like can be stained with the dye to provide water compatibility.

Another aspect of the present invention involves an improvement in an assay for an analyte in a sample suspected of containing said analyte where the analyte is a binding pair member. The method involves a binding pair member conjugated to the dye of the invention.

For example, a fluorescent assay can employ as a reagent a fluorescent compound conjugated to a member of a specific binding pair. Such assay is useful for the determination of an analyte which is also a member of a specific binding pair. The binding of the conjugate to the analyte or a specific binding pair member complex of the analyte is indicative of the presence of the analyte. The present improvement comprises employing in the fluorescent assay a reagent that is a dye conjugated to a member of a specific binding pair. Another example is a method for detecting the presence of a determinant site or a receptor by employing a fluorescent reagent having a fluorescent compound bound to a member of a specific binding pair. The binding of the fluorescent reagent to the determinant site or the receptor, or a specific binding pair member complex of the determinant site or the receptor, is determined as indicative of the presence of the determinant site or the receptor. The method has particular application where the determinant site or receptor is associated with a cell such as, e.g., being present on the cell surface.

The present conjugates can be used for determining qualitatively, semiquantitively or quantitatively an analyte in a sample. Where compounds are to be detected in physiological fluids, the sample may include serum, urine, saliva, lymph or the like. Where the compound of interest is involved in chemical processing or ecological concerns, the sample may be an aqueous medium, or may be obtained by extraction from an organic medium, soil, inorganic mixtures, or the like.

Another reagent in the assay can be a compound of the invention where the binding pair member is a receptor for the analyte.

As previously indicated, the compounds of this invention can include its dyes conjugated to compounds which may be measured by known immunoassay techniques. The conjugates are reagents which compete in an assay medium for the analyte in a sample. Therefore, the conjugate retains a sufficient proportion of the structure of the analyte to be able to compete with the analyte for a receptor for analyte.

The assays may involve a change of spectroscopic properties due to a change in environment about the spectroscopically active compound or the bringing together of a fluorescer-quencher pair within sufficient proximity for the quencher to interact with the fluorescer. Alternatively, methods can be employed which involve the separation of associated and unassociated fluorescer and the detection of the fluorescer in one or both of the fractions.

In carrying out the method an aqueous medium will normally be employed. Other polar solvents may also be employed, usually oxygenated organic solvents of from 1 to 6, more usually from 1 to 4 carbon atoms, including, but not limited to, alcohols, ethers and the like. Usually these cosolvents are present in less than about 40 weight percent, more usually in less than about 20 weight percent.

The pH for the medium usually is in the range of 4 to 11, more usually in the range of 5 to 10, and most preferably in the range of 5.4 to 9.5. The pH is chosen so as to maintain a significant level of binding between binding pair members while optimizing signal producing proficiency. In some instances, a compromise is made between these two considerations. Various buffers may be used to achieve the desired pH and maintain the pH during the determination. Illustrative buffers include borate, phosphate, carbonate, Tris, barbital and the like. The particular buffer employed is not critical to this invention but in individual assays, one buffer may be preferred over another.

Moderate temperatures are normally employed for carrying out the method and usually constant temperatures during the period of the method. The temperatures for the determination will generally range from 10°C to 50°C, more usually from 15°C to 40°C.

The concentration of analyte which may be assayed generally varies from 10⁻⁴ to 10⁻¹⁵ M, more usually from 10⁻⁶ to 10⁻¹³ M. Considerations such as whether the assay is qualitative, semi-quantitative or quantitative, the particular detection technique, and the concentration of the analyte will normally determine the concentration of the other reagents.

While the concentrations of the various reagents are generally determined by the concentration range of interest of the analyte, the final concentration of each of the reagents is normally determined empirically to optimize the sensitivity of the assay over the range of interest. The total binding sites of the members of the specific binding pair which are complementary to the analyte are not less than about 0.1 times the minimum concentration of interest based on binding sites of the analyte and not more than about 10,000 times the maximum concentration of interest based on analyte binding sites, usually about 0.1 to 1000 times, more usually about 0.3 to 10 times the maximum concentration of interest.

The concentration of the compound of the invention in the assay medium is dependent on the type of assay, heterogeneous or homogeneous, competitive or direct, and the like. Normally the compound of the invention is present in the assay medium in a concentration 10⁻⁶ to 10⁻¹⁵, usually 10⁻⁸ to 10⁻¹³ M.

The order of addition of the various reagents may also vary and is dependent on many of the considerations mentioned above.

The present assay method has application both to heterogeneous and homogeneous assays. Exemplary heterogeneous assays are found in U.S. Pat. Nos. 4,256,834 and 4,261,968. Homogeneous immunoassays are exemplified by immunofluorescence methods such as those disclosed in U.S. Pat. No. 3,993,345, enzyme channeling techniques such as those disclosed in U.S. Pat. No. 4,233,402, and other enzyme immunoassays as discussed in U.S. Pat. No. 3,817,837. The assay can be competitive or direct and can involve compounds of the invention that are either labeled ligand or labeled receptor.

In one approach in accordance with the invention for detecting the presence or amount of analyte in a sample suspected of containing said analyte wherein said analyte is a binding pair member consisting of ligand and its complementary receptor the method comprises: (1) combining in an assay medium the sample, as described above, a conjugate of the dye and a binding pair member, and a second binding pair member, wherein the two binding pair members are complementary to the analyte; and (2) determining the effect of said sample on the fluorescence of the binding pair member/dye conjugate as related to the presence or amount of analyte in said sample.

The second binding pair member can be conjugated to a compound capable of quenching the fluorescence of the conjugate when both binding pair members are bound to the analyte. Alternatively, the second binding pair member can be bound to a particle or to a surface or support to permit separation of conjugate that binds to the support from the conjugate remaining in solution.

For example, in one technique, a quencher for the dye is employed. One reagent is a compound of the invention comprising a conjugate of a dye and an analog of a ligand analyte. Another reagent is a conjugate of quencher and a binding pair member that is a receptor for the analyte. The ligand analyte in the sample and the ligand analyte analog in the reagent compete with receptor for analyte. When the receptor for analyte binds to the labeled ligand analyte analog, the fluorescer and quencher are brought within quenching distance. A similar assay employing fluorescent compounds not within the scope of this invention is extensively described in U.S. Pat. No. 3,996,345. The assay technique is described beginning with column 17 and ending at column 23. The ratios of fluorescent compound to ligand and receptor is described in the above-cited patent at columns 4-6.

In a related but different approach one reagent can be a compound of the invention that is a conjugate of a dye and a receptor for the ligand analyte. Another reagent is a conjugate of a quencher for the dye and a receptor for the ligand analyte. When the two reagents above are combined with the sample and brought together by the presence of ligand analyte, the dye and the quencher are brought within quenching distance. A typical quencher can be, e.g., gallocyanine, which can be conjugated through an amide bond to a binding pair member.

The assay is carried out by combining the dye conjugate and the quencher conjugate in conjunction with the sample. The fluorescence is determined in comparison to an assay medium having a known amount of analyte.

In another example the compound of the invention is a conjugate of a dye and a receptor for the ligand analyte. Ligand or ligand analog is bound to a support or to a particle. Similar assays are described in U.S. Pat. No. 4,275,149.

Another approach involves steric exclusion in that receptors for the ligand and for the dye are employed, where simultaneous binding of the receptor for the ligand and receptor for the dye is inhibited. Furthermore, when the receptor for the dye is bound to the dye, the fluorescence of the dye is substantially diminished. Further reduction, if not complete inhibition of fluorescence, can be achieved by conjugation of quencher to receptor for the dye. A similar assay is extensively described in U.S. Pat. No. 3,998,943, issued Dec. 21, 1976. The assay is described in columns 3-5 of the subject patent.

Generally, the method involves combining in an assay medium the sample suspected of containing the analyte, the conjugate of the binding pair member and the dye, and other reagents in accordance with the particular assay protocol chosen. The sample is then exposed to a source of excitation. The fluorescence is determined either as a rate or equilibrium mode. The readings are taken within about 1 second to 1 hour after all materials have been combined for a rate mode, while for an equilibrium mode, the readings may be taken for as long as up to about 24 hours or longer.

As a matter of convenience, the reagents employed in the present invention can be provided in a kit in packaged combination with predetermined amounts of reagents for use in an analyte in a sample. The reagents include a compound of the invention as disclosed above, and, where appropriate, conjugates of quenchers and binding pair members or other reaction partners for the compound of the invention required to provide the detectable signal. In addition, other additives such as ancillary reagents may be included. The relative amounts of the various reagents may be varied widely, to provide for concentrations in solution of the reagents which substantially optimize the sensitivity of the assay. The reagents can be provided as dry powders, usually lyophilized, including excipients, which on dissolution provide for a reagent solution having the appropriate concentrations for performing the assay.

This invention will be further described by the following examples. These are intended to illustrated the invention and are not to be construed as limiting its scope.

### Examples

### Example 1.

The dyes having the molecular structures reported above and a commercial control sample Cy5^{™} (Amersham Biosciences) were dissolved in a solution 1:1 water/methanol.

The UV/Vis absorbance spectra were recorded on a Varian Cary 50 UV spectrophotometer and normalized to a concentration 1µM.

The bleaching study was carried out by taking a sample of each dye, bleaching it in front of a high powered red LED at 633 nm, next to a control solution to monitor for lamp power changes and recording a timecourse of the intensity. Fluorescence emission data were recorded on a Varian Eclipse spectrophotometer using an excitation wavelength of 647 nm. These data are reported in the following Table 2.

**TABLE 2**

| | Absorbance | Fluorescence | Fluorescence Intensity (1µM conc.) | %Fluo intensity retain |
|---|---|---|---|---|
| | λmax (nm) | λmax (nm) | @ 647 nm excitation | t=80' |
| A, invention | 646 | 668 | 902 | 91.2 |
| B, invention | 643 | 666 | 389 | 88.4 |
| C, comparison | 643 | 667 | 576 | 59.9 |
| Cy5^{™}, reference | 649 | 669 | 787 | 76.1 |

The data recorded in table 2 show that the presence of at least one sulfonamide substituent attached to the aromatic ring (compound A and compound B of the invention) improves photostability if compared to analogous dyes with sulfonate groups attached to the aromatic rings (sample Cy5^{™}, Amersham Biosciences) or with no substituents attached to the aromatic rings (sample C).

The improvement in photostability of the samples of the invention is detailed in table 3 (as well as in Figure 1), where the percentage of retained fluorescence intensity is reported against time of irradiation for samples A, B and Cy5^{™}.

**TABLE 3**

| Time (minutes) | % of Retained Fluo Intensity | | |
|---|---|---|---|
| | Cy5^{™} | Sample A | Sample B |
| 0 | 100.0 | 100.0 | 100.0 |
| 10 | 101.4 | 100.6 | 104.1 |
| 20 | 90.6 | 96.6 | |
| 45 | | | 98.7 |
| 80 | 76.1 | 91.2 | 88.4 |
| 140 | 67.7 | | |
| 185 | | | 72.3 |
| 200 | 54.3 | | |
| 290 | 38.4 | 90.0 | |
| 310 | | | 61.9 |
| 350 | 29.9 | 88.9 | |
| 420 | 24.1 | 88.1 | |

The data reported above show that the introduction of at least one sulfonamide substituent attached to the aromatic ring improve the photostability of the dye when compared to reference dye. This improvement in photostability is retained also after conjugation with the substrate. This property makes this class of fluorescent dyes particularly useful as fluorescent markers for biological, medical or analytical applications.

### Example 2.

As described in previous Example 1, but utilizing this time the dyes B, D and E of the invention, having molecular structures reported above, and the commercial control sample Cy5^{™} (Amersham Biosciences).

The data are reported in the following Table 4, measuring the percentage of retained fluorescence intensity after 2 hours.

**TABLE 4**

| | Absorbance | Fluorescence | Fluorescence Intensity (1µM conc.) | %Fluo intensity retain |
|---|---|---|---|---|
| | λmax (nm) | λmax (nm) | @ 647 nm excitation | t=2 hours |
| B, invention | 643 | 666 | 389 | 86.7 |
| D, invention | 649 | 669 | 650 | 84.9 |
| E, invention | 646 | 668 | 800 | 90.2 |
| Cy5^{™}, reference | 649 | 669 | 787 | 73.2 |

The data recorded in Table 4 show that the presence of at least one sulfonamide substituent attached to the aromatic ring (compound B, D and E of the invention) improves photostability if compared to analogous dyes with only sulfonate groups attached to the aromatic rings (sample Cy5^{™}, Amersham Biosciences).
The improvement in photostability of the samples of the invention is detailed in Table 5 (as well as in Figure 2), where the percentage of retained fluorescence intensity is reported against time of irradiation for samples B, D and Cy5^{™}.

**TABLE 5**

| Time (minutes) | % of Retained Fluo Intensity | | | |
|---|---|---|---|---|
| | Cy5^{™} | Sample B | Sample D | Sample E |
| 0 | 100.0 | 100.0 | 100.0 | 100.0 |
| 60 | 85.3 | 90.7 | 92.1 | 91.0 |
| 120 | 73.2 | 86.7 | 84.9 | 90.2 |
| 180 | 62.5 | 87.4 | 80.7 | 89.5 |
| 240 | 53.0 | 80.8 | 74.9 | 88.8 |
| 420 | 30.0 | 71.4 | 57.7 | 87.6 |

The data reported above show that the introduction of at least one sulfonamido substituent attached to the aromatic ring improves the photostability of the dye when compared to reference dye. This improvement in photostability is retained also after conjugation with the substrate. This property makes this class of fluorescent dyes particularly useful as fluorescent markers for biological, medical or analytical applications.

## Claims

1. A fluorescent cyanine dye having the formula (I) 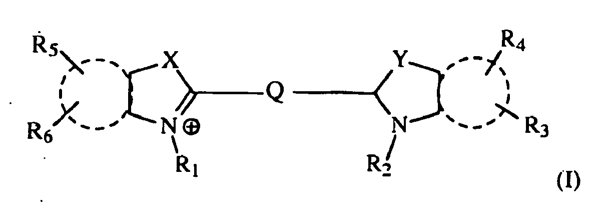 wherein:
X and Y are both -C(CH₃)₂;
the dotted curves independently represent the carbon atoms required to form a benzo-condensed or a naphtho-condensed ring;
R₁ and R₂ each independently represents sulfoalkyl, carboxyalkyl or sulfatoalkyl groups, wherein the alkyl chain of said R₁ and R₂ groups comprises from 1 to 5 carbon atoms;
R₃, R₄, R₅ and R₆ each independently is hydrogen atom, halogen atom, alkyl group, alkoxy group, amino group, and sulfo group;
**characterized in that**
at least one of R₃, R₄, R₅ and R₆ is a -SO₂NR₇R₈ group wherein R₇ and R₃ each independently represents hydrogen, unsubstituted alkyl, aryl, or -COCH₃, with the proviso that when R₇ is -COCH₃, R₃ is hydrogen;
Q is a polymethine chain having from 3 to 7 carbon atoms selected from the group consisting of: 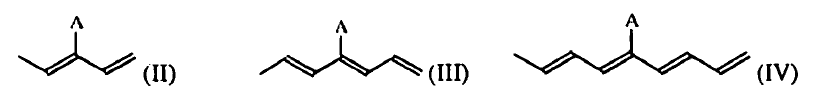 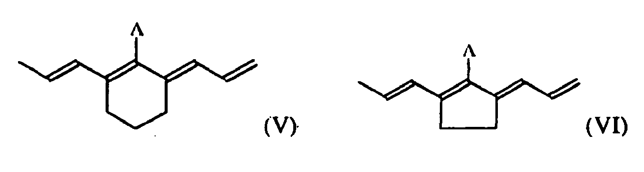 wherein A represents hydrogen, halogen, alkyl, aryl, cyano, -N(R₉R₁₀) where R₉ and R₁₀ each independently represents alkyl, aryl or together represent the non-metal atoms necessary to form a 5- or 6-membered heterocyclic ring, or -Z-R₁₁ where Z is O or S, and R₁₁ represents alkyl or aryl;
at least one of R₁, R₂, A comprises a substituent selected from the group consisting of succinimidyl, anhydrides, acid halides, isothiocyanate, vinylsulphone, dichlorotriazines, haloacetamides, maleimides, carboxyl, phosphoramidites.

2. The fluorescent cyanine dye of claim 1, wherein said R₇ and R₈ are both hydrogen.

3. The fluorescent cyanine dye of claim 1, wherein said 5- or 6-membered heterocyclic ring is selected from the group consisting of piperidine, piperazine, piperimidine, piperidazine, morpholine, pyrrolidine, imidazole and triazole.

4. A labeling composition comprising a fluorescent cyanine dye according to any one of claims 1 to 3.

5. A labeling reagent for labeling a target, the labeling reagent comprising a fluorescent cyanine dye according to any one of claims 1 to 3.

6. An element selected within the group consisting in nucleotides, nucleosides, nucleic acid, polynucleotides and polypeptides, said element being labeled with a fluorescent cyanine dye according to any one of claims 1 to 3.

7. A method for labeling an element selected within the group consisting in nucleotides, nucleosides, nucleic acid, polynucleotides and polypeptides, said method comprising contacting said element with a fluorescent cyanine dye according to any one of claims 1 to 3.

8. A method for determining an analyte, **characterized in that** said analyte is a binding pair member and wherein the other member of said pair is conjugated to a fluorescent cyanine dye according to any one of claims 1 to 3.

## Patentansprüche

1. Fluoreszierender Cyaninfarbstoff mit der Formel (I) 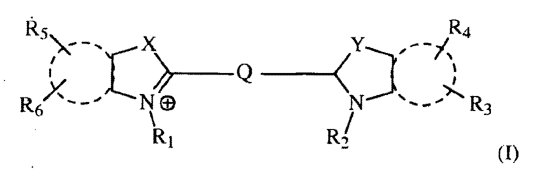 wobei:
X und Y beide -C(CH₃)₂ sind;
die punktierten Kurven unabhängig die Kohlenstoffatome darstellen, welche erforderlich sind, um einen benzo-kondensierten oder naphto-kondensierten Ring zu bilden;
R₁ und R₂ jeweils unabhängig Sulfoalkyl-, Carboxyalkyl- oder Sulfatoalkylgruppen darstellen, wobei die Alkylkette der R₁- und R₂-Gruppen 1 bis 5 Kohlenstoffatome aufweist;
R₃, R₄, R₅ und R₆ jeweils unabhängig ein Wasserstoffatom, Halogenatom, Alkylgruppe, Alkoxygruppe, Aminogruppe und Sulfogruppe ist;
**dadurch gekennzeichnet,**
**dass** zumindest einer der R₃, R₄, R₅ und R₆ eine -SO₂NR₇R₈-Gruppe ist, wobei R₇ und R₈ jeweils unabhängig Wasserstoff, unsubstituierter Alkyl-, Aryl- oder -COCH₃ ist, mit der Bedingung, dass R₇ -COCH₃ ist, R₈ Wasserstoff ist;
Q eine Polymethinkette mit 3 bis 7 Kohlenstoffatomen ist, ausgewählt aus der Gruppe bestehend aus: 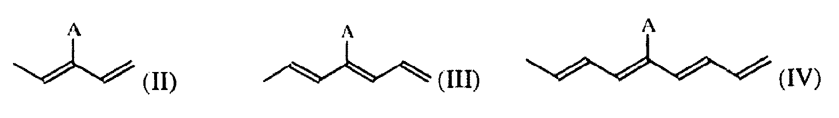 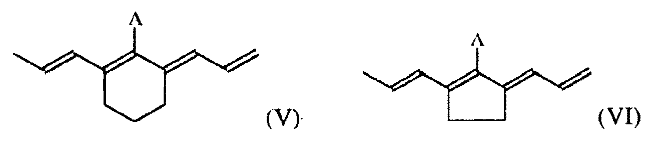 wobei A Wasserstoff, Halogen, Alkyl, Aryl, Cyano, -N(R₉R₁₀) darstellt, wobei R₉ und R₁₀ jeweils unabhängig Alkyl, Aryl darstellen oder gemeinsam die Nicht-Metallatome darstellen, welche notwendig sind, um einen 5 oder 6-atomigen heterozyklischen Ring oder -Z-R₁₁ zu bilden, wobei Z O oder S ist und R₁₁ Alkyl oder Aryl darstellt;
zumindest einer von R₁, R₂, A einen Substituenten aufweist, der ausgewählt ist aus der Gruppe bestehend aus Succinimidyl, Anhydriden, Säurehalogeniden, Isothiocyanaten, Vinylsulphon, Dichlortriazinen, Haloacetamiden, Maleimiden, Carboxyl, Phosphoramiditen.

2. Fluoreszierender Cyaninfarbstoff nach Anspruch 1,
wobei sowohl R₇ und R₈ Wasserstoff sind.

3. Fluoreszierender Cyaninfarbstoff nach Anspruch 1,
wobei der 5- oder 6-atomige heterozyklische Ring ausgewählt ist aus der Gruppe bestehend aus Piperidin, Piperazine, Piperimidin, Piperidazin, Morpholin, Pyrrolidin, Imidazol und Triazol.

4. Markierungszusammensetzung mit einem fluoreszierenden Cyaninfarbstoff nach einem der Ansprüche 1 bis 3.

5. Markierungsreagenz zum Markieren eines Zieles,
wobei das Markierungsreagenz einen fluoreszierenden Cyaninfarbstoff nach einem der Ansprüche 1 bis 3 enthält.

6. Element ausgewählt aus der Gruppe bestehend aus Nucleotiden, Nucleosiden, Nucleinsäuren, Polynucleotiden und Polypeptiden,
wobei das Element mit einem fluoreszierenden Cyaninfarbstoff nach einem der Ansprüche 1 bis 3 markiert ist.

7. Verfahren zum Markieren eines Elementes ausgewählt aus der Gruppe bestehend aus Nucleotiden, Nucleosiden, Nucleinsäure, Polynucletiden und Polypeptiden, wobei das Verfahren aufweist, das Element mit einem fluoreszierenden Cyaninfarbstoff nach einem der Ansprüche 1 bis 3 zu kontaktieren.

8. Verfahren zum Ermitteln eines Analyten,
**dadurch gekennzeichnet,**
**dass** der Analyt ein Bindungspaarpartner ist, und wobei der andere Partner des Paares mit einem fluoreszierenden Cyaninfarbstoff nach einem der Ansprüche 1 bis konjugiert ist.

## Revendications

1. Colorant cyanine fluorescent répondant à la formule (I) 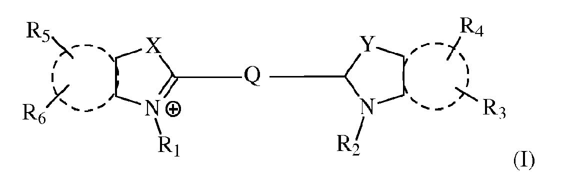 dans laquelle :
X et Y représentent tous deux un groupe -C(CH₃)₂ ;
les courbes en pointillés représentent indépendamment les atomes de carbone nécessaires pour former un noyau benzo-condensé ou naphto-condensé ;
R₁ et R₂ représentent chacun indépendamment un groupe sulfoalkyle, carboxyalkyle ou sulfatoalkyle, dans laquelle la chaîne alkyle desdits groupes R₁ et R₂ comprend de 1 à 5 atomes de carbone ;
R₃, R₄, R₅ et R₆ représentent chacun indépendamment l'atome d'hydrogène, un atome d'halogène, un groupe alkyle, un groupe alcoxy group, un groupe amino group et un groupe sulfo ;
**caractérisé en ce que**
au moins l'un de R₃, R₄, R₅ et R₆ représente un groupe -SO₂NR₇R₈ dans lequel R₇ et R₈ représentent chacun indépendamment l'atome d'hydrogène, un groupe alkyle non substitué, un groupe aryle ou un groupe -COCH₃, à condition que lorsque R₇ représente un groupe -COCH₃, R₈ représente l'atome d'hydrogène ;
Q représente une chaîne de polyméthine comportant de 3 à 7 atomes de carbone choisie dans le groupe constitué de : 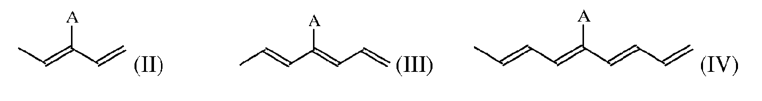 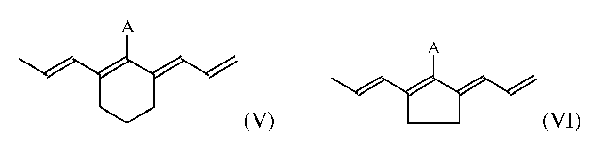
formules dans lesquelles A représente l'atome d'hydrogène, un atome d'halogène, un groupe alkyle, un groupe aryle, un groupe cyano, un groupe -N(R₉R₁₀) où R₉ et R₁₀ représentent chacun indépendamment un groupe alkyle, un groupe aryle ou représentent conjointement les atomes non métalliques nécessaires pour former un noyau hétérocyclique à 5 ou 6 chaînons, ou un groupe -Z-R₁₁ où Z représente un atome d'O ou de S, et R₁₁ représente un groupe alkyle ou aryle ;
au moins l'un de R₁, R₂, A comprend un substituant choisi dans le groupe constitué du groupe succinimidyle, des anhydrides, des halogénures d'acide, de l'isothiocyanate, de la vinylsulfone, des dichlorotriazines, des halogénoacétamides, des maléimides, du carboxyle, des phosphoramidites.

2. Colorant cyanine fluorescent selon la revendication 1, dans lequel lesdits R₇ et R₈ sont tous deux un atome d'hydrogène.

3. Colorant cyanine fluorescent selon la revendication 1, dans lequel ledit noyau hétérocyclique à 5 ou 6 chaînons est choisi dans le groupe constitué de la pipéridine, de la pipérazine, de la pipérimidine, de la pipéridazine, de la morpholine, de la pyrrolidine, de l'imidazole et du triazole.

4. Composition de marquage comprenant un colorant cyanine fluorescent selon l'une quelconque des revendications 1 à 3.

5. Réactif de marquage permettant de marquer une cible, le réactif de marquage comprenant un colorant cyanine fluorescent selon l'une quelconque des revendications 1 à 3.

6. Élément choisi dans le groupe constitué des nucléotides, des nucléosides, des acides nucléiques, des polynucléotides et des polypeptides, ledit élément étant marqué par un colorant cyanine fluorescent selon l'une quelconque des revendications 1 à 3.

7. Procédé permettant de marquer un élément choisi dans le groupe constitué des nucléotides, des nucléosides, des acides nucléiques, des polynucléotides et des polypeptides, ledit procédé comprenant la mise en contact dudit élément avec un colorant cyanine fluorescent selon l'une quelconque des revendications 1 à 3.

8. Procédé de détermination d'un analyte, **caractérisé en ce que** ledit analyte est un membre d'une paire de liaison et dans lequel l'autre membre de ladite paire est conjugué à un colorant cyanine fluorescent selon l'une quelconque des revendications 1 à 3.
